(19) Europäisches Patentamt
European Patent Office
Office européen des brevets

(11) Veröffentlichungsnummer: **0 002 181**
**B1**

(12) # EUROPÄISCHE PATENTSCHRIFT

(45) Veröffentlichungstag der Patentschrift: **22.07.81**

(21) Anmeldenummer: **78101082.2**

(22) Anmeldetag: **06.10.78**

(51) Int. Cl.³: **C 07 D 209/48,**
C 07 C 117/08 //C08F8/30

(54) **3- und 4-Azidophthalsäurederivate und Verfahren zu ihrer Herstellung.**

(30) Priorität: **14.10.77 CH 12577/77**

(43) Veröffentlichungstag der Anmeldung:
**13.06.79 Patentblatt 79/12**

(45) Bekanntmachung des Hinweises auf die Patenterteilung:
**22.07.81 Patentblatt 81/29**

(84) Benannte Vertragsstaaten:
**CH DE FR GB NL SE**

(56) Entgegenhaltungen:
**GB - A - 843 541**
**GB - A - 1 309 959**

(73) Patentinhaber: **CIBA-GEIGY AG**
**Patentabteilung Postfach**
**CH-4002 Basel (CH)**

(72) Erfinder: **Kvita, Vratislav, Dr.**
**St. Jakobstrasse 2**
**CH-4132 Muttenz (CH)**
Erfinder: **Zweifel, Hans, Dr.**
**Lothringerstrasse 93**
**CH-4056 Basel (CH)**
Erfinder: **Greber, Gerd, Prof. Dr.**
**Anzengruberstrasse 11**
**A-2540 Bad Vöslau (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 181 B1

Courier Press, Leamington Spa, England.

# 3- und 4-Azidophthalsäurederivate und Verfahren zu ihrer Herstellung

Die vorliegende Erfindung betrifft neue 3- oder 4-Azidophthalsäurederivate und Verfahren zu deren Herstellung. Die erfindungsgemässen 3- und 4-Azidophthalsäurederivate eignen sich zur Herstellung von unter der Einwirkung von Licht vernetzbaren Polymeren.

Aus der Literatur ist bekannt, dass sich Polymere mit seitenständigen Azidogruppen unter Lichteinwirkung vernetzen lassen und sich für photomechanische Anwendungen eignen [vgl. z.B. britische Patentschrift 843.541, US Patentschrift 3.002.003; Journal of Appl.Poly.Sci.,7,273—279 (1963) und japanische Offenlegungsschrift 74/23843]. Diese vorbekannten Polymeren sind insofern nachteilig, als sie vorwiegend im kurzwelligen UV—Bereich absorbieren und sich daher nicht oder nur schlecht für zahlreiche Anwendungen, besonders auf dem Gebiet der Mikroelektronik, eignen, die hoch lichtempfindliche und im längerwelligen UV—Bereich absorbierende Substanzen erfordern.

Aufgabe der Erfindung war daher die Bereitstellung von neuen Substanzen, die sich zur Herstellung von Polymeren mit erhöhter Lichtempfindlichkeit eignen, die im längerwelligen UV—Bereich (über 320 nm) absorbieren.

Die neuen 3- und 4-Azidophthalsäurederivate entsprechen der Formel Ia oder Ib

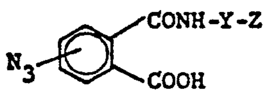

oder

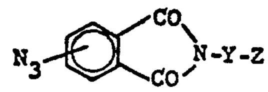

(Ia) (Ib),

worin Y unsubstituiertes oder substituiertes Alkylen mit 2—18 C—Atomen, eine unsubstituierte oder substituierte Phenylen-, Naphthylen-, Biphenylen-, Cyclohexylen-, Dicyclohexylmethangruppe oder eine unsubstituierte oder substituierte Gruppierung

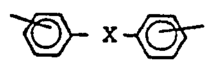

X —O—, —S—, $—SO_2—$, $—CH_2—$, $—CH(CH_3)—$, $—C(CH_3)_2—$ oder —CO— und

Z $—NH_2$, —NH—Alkyl mit 1—4 C—Atomen, —OH, —COOH, —COCl, $—O—CO—CH{=}CH_2$, $—O—CO—C(CH_3){=}CH_2$, $—COO—CH{=}CH_2$ oder $—O—CH{=}CH_2$ darstellen.

Durch Y dargestellte Alkylengruppen können geradkettig oder verzweigt und gegebenenfalls substituiert sein, z.B. durch eine oder mehrere Phenylgruppen, Cycloalkylgruppen mit 5—8 C—Atomen oder Aralkylgruppen mit 7 oder 8 C—Atomen. Unter den substituierten Alkylengruppen Y sind solche bevorzugt, die durch eine oder zwei Phenylgruppen oder durch eine oder zwei definitionsgemässe Cycloalkyloder Aralkylgruppen, wie die Cyclohexyl- oder die Benzylgruppe, substituiert sind.

Beispiele von derartigen Alkylengruppen Y sind: die 1,2-Aethylen-, 1,3- oder 1,2-Propylen, 1,4- oder 1,3-Butylen-, 1,5-Pentamethylen-, 1,6-Hexamethylen-, 2-Methyl-4-dimethylhexan-, 2-Dimethyl-4-methylhexan-, 1,10-Dicyclohexyl- oder 1,10-Dicyclooctyl-1,10-decan-, 1,10-Di-isopropyldecan-, 1,1,10,10-Tetramethyldecan-, 1,10-Diäthyl-1,10-dimethyldecan-, 1,8-Octamethylen-, 1,10-Decamethylen-, 1,12-Dodecamethylen- und 1-Aethyl-10,10-dimethylundecamethylengruppe. Bevorzugt sind unsubstituierte geradkettige oder verzweigte Alkylengruppen, besonders solche mit 2—16 C—Atomen.

Durch Y dargestellte Phenylen-, Naphthylen-, Diphenylen-, Cyclohexylen- oder Dicyclohexylmethangruppen oder Gruppierungen Y

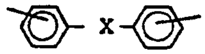

können ebenfalls substituiert sein, z.B. durch Halogenatome, wie Fluor, Chlor oder Brom, Alkylgruppen mit 1—4 C—Atomen, besonders Methyl oder Aethyl, Cycloalkylgruppen mit 5—7 C—Atomen, besonders Cyclopentyl und Cyclohexyl, oder Aralkylgruppen mit 7 oder 8 C—Atomen, wie Benzyl oder β-Phenyläthyl. Die genannten Gruppen können an jedem Ring mehrere Substituenten der erwähnten Art aufweisen, sind aber mit Vorteil pro Ring nur durch eine der genannten Gruppen substituiert, vor allem durch Chlor oder Brom, Methyl oder Aethyl.

Bevorzugt sind Phenylen-, Naphthylen-, Cyclohexylen-, Biphenylen-, Dicyclohexylmethan- und

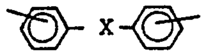

Gruppen Y jedoch unsubstituiert, und X stellt mit Vorteil —O—, —$SO_2$— oder —$CH_2$— dar. Besonders bevorzugt sind Cyclohexylen-, Naphthylen- und vor allem Phenylengruppen.

Die $N_3$-Gruppe ist bevorzugt in ortho-Stellung zur Carboxyl- oder Carbonamidgruppe bzw. zu einer Carbonylgruppe an den Benzolring gebunden.

Bevorzugt sind Verbindungen der Formel Ia und besonders solche der Formel Ib, worin Y unsubstituiertes geradkettiges oder verzweigtes Alkylen darstellt und Z die unter Formel Ia bzw. Ib angegebene Bedeutung hat, vor allem aber Verbindungen der Formel Ia und besonders Verbindungen der Formel Ib, worin Y unsubstituiertes Alkylen mit 2—18, besonders 2—16 C—Atomen, Cyclohexylen, Naphthylen oder Phenylen und Z —$NH_2$, —COOH, —COCl, —OH, —O—CO—CH=$CH_2$ oder

—O—CO—C=$CH_2$
|
$CH_3$

darstellen.

Besonders bevorzugte erfindungsgemässe Produkte sind Verbindungen der Formel VII

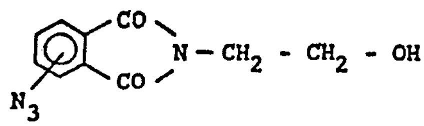 (VII),

vorzugsweise das N-(β-Hydroxyäthyl)-3-azidophthalimid, und Verbindungen der Formel VIII

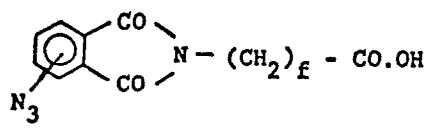 (VIII),

worin f eine Zahl von 2 bis 5, vorzugsweise 5 bedeutet, vorzugsweise die 6-(3-Azidophthalsäureimidyl)-capronsäure, und Verbindungen der Formel IX

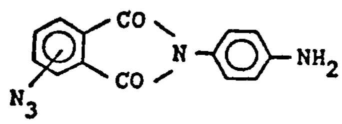 (IX),

vorzugsweise das 4-(3-Azidophthalsäureimidyl)-anilin, und Verbindungen der Formel X

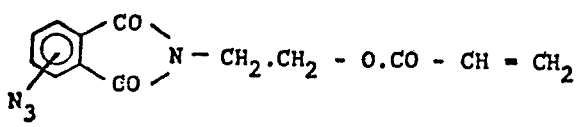 (X)

und Verbindungen der Formel XI

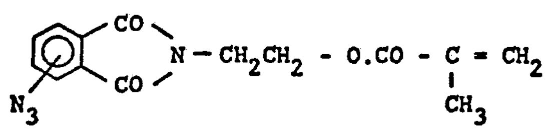 (XI)

Die Verbindungen der Formel Ib können auf besonders vorteilhafte Weise dadurch hergestellt werden, dass man eine Verbindung der Formel II

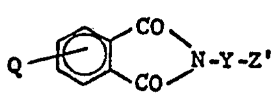 (II),

worin Y die unter Formel Ib angegebene Bedeutung hat, Z' eine Z entsprechende Gruppierung darstellt, aber ungleich —COCl ist und Q ein Halogenatom, wie Chlor, Brom oder Fluor, oder die Nitrogruppe bedeutet, in einem inerten organischen Lösungsmittel bei einer Temperatur zwischen etwa 0°C und 120°C, bevorzugt zwischen etwa 50°C und 90°C, mit einem Azid der Formel III

$$M^{n+}(N_3^-)_n \quad \text{(III)}$$

umsetzt, worin n die Zahl 1 oder 2 und M ein Alkalimetall-, Erdalkalimetall- oder quaternäres Ammoniumkation bedeuten, und die erhaltene Verbindung der Formel Ib, worin Z —COOH darstellt, gegebenenfalls durch Behandeln mit einem geeigneten Chlorierungsmittel, wie Thionylchlorid, Oxalylchlorid oder Phosgen, in das entsprechende Säurechlorid überführt.

Aus der Literatur [Tetrahedron Letters, *12*, 1305—1309 (1966)] ist bekannt, dass sich 3-Nitrophthalsäureanhydrid bei einer Temperatur von 110°C mit Natriumazid in Gegenwart eines inerten organischen Lösungsmittels in das 3-Azidophthalsäureanhydrid überführen lässt. Das 4-Nitrophthalsäureanhydrid und das 3-Chlorphthalsäureanhydrid lassen sich jedoch nach dieser Methode nicht in das entsprechende Azid überführen. Es ist daher überraschend, dass sich erfindungsgemäss sowohl 3-Nitro- als auch 4-Nitrophthalimide der Formel II und die entsprechenden 3- und 4-Halogenverbindungen zu den entsprechenden Aziden umsetzen lassen und die Reaktion zudem in den meisten Fällen bei wesentlich niedrigeren Temperaturen mit guten bis sehr guten Ausbeuten durchgeführt werden kann. Eine vorherige aufwendige Auftrennung der Ausgangsprodukte in die 3- und 4-Isomeren ist daher beim erfindungsgemässen Verfahren nicht notwendig.

Q stellt bevorzugt die Nitrogruppe dar. Stellt M ein quaternäres Ammoniumkation dar, so handelt es sich z.B. um ein Tetraalkyl- oder Benzyltrialkylammoniumkation mit je 1—12 und insbesondere 1—4 C—Atomen in den Alkylteilen, insbesondere das Tetramethyl- oder Trimethylbenzylammoniumkation.

Beispiele von geeigneten Alkalimetall- oder Erdalkalimetallaziden sind das Lithium-, Natrium-, Kalium-, Calcium-, Magnesium- und Bariumazid. Bevorzugt verwendet man Alkalimetallazide, vor allem das Natriumazid. Das Azid wird zweckmässig im Ueberschuss eingesetzt, beispielsweise einem etwa 5—50%igen und bevorzugt einem etwa 10—30%igen molaren Ueberschuss.

Als inerte organische Lösungsmittel kommen z.B. polare Lösungsmittel in Betracht, beispielsweise:

— niedere aliphatische Alkohole, z.B. solche mit bis zu 6 C—Atomen, wie Methanol, Aethanol, Propanol, Isopropanol, Butanole und Pentanole.

— Dibenzyläther und Dialkyläther mit je 1—4 C—Atomen in den Alkylteilen, wie Diäthyläther, Di-n-propyläther und und Di-isopropyläther;

— cyclische Aether, wie Tetrahydrofuran, Tetrahydropyran und Dioxan;

— Diäthylenglykol- und Triäthylenglykoldialkyläther mit je 1—4 C—Atomen in den Alkylteilen, wie Diäthylenglykoldiäthylund -di-n-butyläther sowie Triäthylenglykoldimethyläther.

Mit Vorteil verwendet man aprotische polare Lösungsmittel, wie aliphatische und aromatische Nitrile, wie Alkylnitrile mit 2—5 C—Atomen im Alkylteil, z.B. Acetonitril, Propionitril, Butyronitril, oder Benzonitril; cyclische Amide, wie N-Methyl-2-pyrrolidon, N-Acetyl-2-pyrrolidon und N-Methyl-ε-caprolactam; N,N-Dialkylamide von aliphatischen Monocarbonsäuren mit 1—3 C—Atomen im Säureteil, wie N,N-Dimethylformamid, N,N-Dimethylacetamid, N,N-Diäthylacetamid und N,N-Dimethylmethoxyacetamid; Dialkylsulfoxide, wie Dimethyl- und Diäthylsulfoxid; Hexamethylphosphorsäuretriamid (Hexametapol); Tetrahydrothiophendioxid (Sulfolan). Bevorzugte Lösungsmittel sind Dialkylsulfoxide, besonders Dimethylsulfoxid.

Verbindungen der Formel Ia und Ib, worin Z ≠ $—OCH{=}CH_2$, können auch dadurch hergestellt werden, dass man eine Verbindung der Formel IV

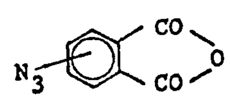

(IV)

mit einem Amin der Formel V $H_2N—Y—Z''$ (V)

zu einer Verbindung der Formel VI

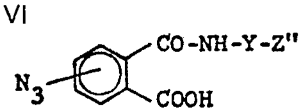

(VI)

umsetzt, wobei Y die unter Formel Ia bzw. Ib angegebene Bedeutung hat und Z″ $—NH_2$, —NH—Alkyl mit 1—4 C—Atomen, —OH, —COOH, $—O—CO—CH{=}CH_2$, $—O—CO—C(CH_3){=}CH_2$ oder $—COO—CH{=}CH_2$ darstellt, die Verbindung der Formel VI gegebenenfalls anschliessend cyclisiert und Verbindungen der Formel Ib, worin Z —COOH darstellt, gewünschtenfalls durch Behandeln mit einem Chlorierungsmittel in das entsprechende Säurechlorid überführt.

Die Umsetzung der Anhydride der Formel IV mit den Aminen der Formel V wird zweckmässig in organischem Medium vorgenommen, wobei je nach Art der Reaktionskomponenten bei Temperaturen zwischen etwa 0°C und 120°C gearbeitet wird. Zweckmässig setzt man das Anhydrid der Formel IV in stöchiometrischer Menge oder einem leichten Ueberschuss über das Amin der Formel V ein, z.B. in einem bis zu etwa 20%igen molaren Ueberschuss.

Beispiele geeigneter organischer Lösungsmittel sind aprotische Lösungsmittel der vorerwähnten

Art, sowie gegebenenfalls halogenierte aliphatische oder aromatische Kohlenwasserstoffe, wie Methylenchorid, Dichlormethan, Chloroform, Tetrachlorkohlenstoff, 1,1,2-Trichloräthan, 1,2-Dichloräthan, Benzol, Toluol and Chlorbenzol, sowie aliphatische und cycloaliphatische Ketone, wie Aceton, Methyläthylketon, Cyclopentanon und Cyclohexanon.

Die Cyclisierung der Verbindungen der Formel VI kann auf an sich bekannte Weise chemisch, d.h. unter Zusatz an sich bekannter Dehydratisierungsmittel, vorgenommen werden. Je nach Art der Reaktionskomponenten, der Reaktionsbedingungen und des eingesetzten Lösungsmittels kann die Cyclisierung, besonders bei erhöhten Temperaturen, auch ohne Zusatz von Dehydratisierungsmitteln erfolgen, wobei das gebildete Wasser zweckmässig azeotrop entfernt wird. Im allgemeinen wird die Cyclisierung jedoch bei Temperaturen zwischen etwa 40 und 120°C, bevorzugt 70—90°C in Gegenwart von Dehydratisierungsmitteln und gegebenenfalls in Gegenwart eines aprotischen organischen Lösungsmittels durchgeführt. Als Dehydratisierungsmittel kommen vor allem Anhydride von gegebenenfalls durch Halogenatome oder Alkylgruppen substituierten aliphatischen Monocarbonsäuren mit 2—5 C—Atomen in Betracht, wie Essigsäure-, Propionsäure-, Buttersäure- und Valeriansäureanhydrid, Trichlor- und Trimethylessigsäureanhydrid. Bevorzugtes Dehydratisierungsmittel ist Essigsäureanhydrid.

Verbindungen der Formel Ia oder Ib, worin Z $-O-CO-CH=CH_2$ oder $-O-CO-C(CH_3)=CH_2$ darstellt, können auch dadurch erhalten werden, dass man eine Verbindung der Formel Ia oder Ib, worin Z —OH ist, mit entsprechenden ungesättigten Säuren, Säurechloriden oder Estern umsetzt. Schliesslich können Verbindungen der Formel Ia oder Ib, worin Z $-COOCH=CH_2$ bedeutet, auch durch Umsetzung von Verbindungen der Formel Ia oder Ib, worin Z —COOH bedeutet, mit entsprechenden Alkoholen oder Estern in Gegenwart von Säuren oder Basen erhalten werden.

Die Ausgangsprodukte der Formeln II bis V sind bekannt oder können auf an sich bekannte Weise hergestellt werden. Verbindungen der Formel II können z.B. auf an sich bekannte Weise durch Umsetzen von 3- oder 4-Nitrophthalsäureanhydrid oder der entsprechenden Halogenverbindungen mit Aminen H N—Y—Z' und anschliessende Cyclisierung der dabei gebildeten Amidcarbonsäuren erhalten werden. Die Azidophthalsäureanhydride der Formel IV sind in der US Patentschrift 3.002.003 beschrieben.

Nach beendeter Umsetzung können die Verbindungen der Formel Ia oder Ib auf übliche Weise gereinigt und isoliert werden, z.B. durch Einengen der Reaktionslösung bzw. -suspension im Vakuum und Waschen des Reaktionsproduktes mit Wasser. Die Verbindungen der Formel Ia und Ib fallen im allgemeinen in Form von Kristallen an.

Die Verbindungen der Formel Ib stellen wertvolle Zwischenprodukte zur Herstellung von lichtvernetzbaren Polymeren dar. Derartige Polymere lassen sich nach an sich bekannten Synthesemethoden zur Herstellung von Makromolekülen mit photoaktiven seitenständigen Gruppen herstellen. Grundsätzlich kommen zwei Methoden in Betracht:

1. Einbau der Azidophthalimidylgruppen in eine bestehende Polymerkette mit entsprechenden funktionellen Gruppen;
2. Aufbau der Polymerkette aus Monomeren, welche die lichtempfindliche Azidophthalimidylgruppierung bereits enthalten, wobei die Polymerkette durch Polymerisation oder Polyaddition aufgebaut werden kann.

Für das Verfahren 1) eignen sich z.B. Verbindungen der Formel Ib, worin Z —OH, —COOH, —COCl, $-NH_2$ oder —NH—Alkyl mit 1—4 C—Atomen bedeutet, indem man derartige Verbindungen beispielsweise mit Polymeren mit wiederkehrenden Strukturelementen der Formeln

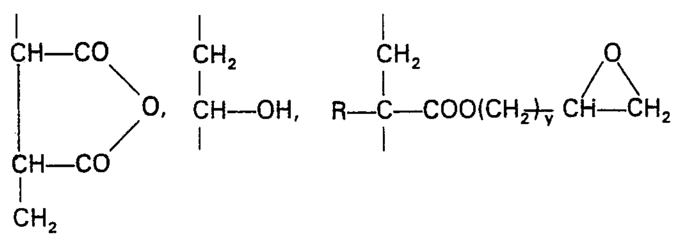

oder

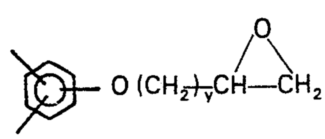

umsetzt (R = Wasserstoff oder Methyl, y = 1 oder 2).

Fur das Verfahren 2) eignen sich vor allem Verbindungen der Formel Ib, worin Z $-O-COCH=CH_2$, $-O-COC(CH_3)=CH_2$, $-COOCH=CH_2$ oder $-OCH=CH_2$ darstellt, indem man derartige Verbindungen allein oder zusammen mit anderen äthylenisch ungesättigten Comonomeren, z.B. Vinylchlorid, Vinylidenchlorid, Acrylsäure,

Acrylnitril, Acrylsäure- und Methacrylsäurealkylestern, Styrol, Aethylen, Propylen, Isopren, Chloropren, 1,4-Butadien, Essigsäure-oder Propionsäurevinylestern, Malein- oder Fumarsäure oder Maleinsäureanhydrid, polymerisiert.

Die so erhaltenen Polymeren mit seitenständigen Azidophthalimidylgruppen lassen sich unter der Einwirkung von Licht, besonders UV—Licht, vernetzen und eignen sich für photomechanische Anwendungen, beispielsweise zur Herstellung von Druckplatten für das Offsetdruckverfahren zur Herstellung von Photooffset-Lacken, für die unkonventionelle Photographie, z.B. zur Herstellung von sogenannten Vesikularbildern oder zum Anfärben von nach dem Belichten und Entwickeln schlecht sichtbaren Polymerbildern mit geeigneten Farbstoffen, wie öllösliche Farbstoffe oder, wenn das Polymere saure Gruppen, wie Carbonsäure- oder Sulfonsäuregruppen aufweist, kationische Farbstoffe. Solche Polymere finden insbesondere Anwendung als sogenannte Photoresist zur Herstellung von gedruckten Schaltungen nach an sich bekannten Methoden. Dabei wird die mit der lichtempfindlichen Schicht versehene Seite der Leiterplatte durch ein das Leiterbild aufweisendes Dianegativ belichtet und dann entwickelt, worauf man die unbelichteten Stellen der Schicht durch Entwicklungsflüssigkeit herausholt. Die Belichtung kann mit Sonnenlicht, Kohlelichtbogen oder Xenonlampen durchgeführt werden. Mit Vorteil wird die Belichtung mit Quecksilberhochdrucklampen vorgenommen.

Beispiel 1

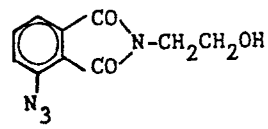

Ein Gemisch von 17,9 g (0,076 Mol) N-(β-Hydroxyäthyl)-3-nitrophthalimid und 5,11 g (0,078 Mol) Natriumazid in 70 ml Dimethylsulfoxid wird bei 50°C während 12 Stunden gerührt. Die Lösung wird im Vakuum eingedampft und mit 200 ml Eiswasser verrührt. Die ausgefallenen Kristalle werden abgesaugt, mit 20 ml Wasser nachgewaschen und 24 Stunden bei 80°C/100 Torr getrocknet. Man erhält 17 g (97% d.Th.) N-(β-Hydroxyäthyl)-3-azidophthalimid; Fp. 141°C (Zersetzung). IR—Spektrum ($CH_2Cl_2$): 1775 $cm^{-1}$ und 1720 $cm^{-1}$ (CO—N—CO); 2120 $cm^{-1}$ ($N_3$).

Das im obigen Beispiel verwendete N-(β-Hydroxyäthyl-3-nitrophthalimid kann wie folgt hergestellt werden: Ein Gemisch von 19,3 g (0,1 Mol) 3-Nitrophthalsäureanhydrid, 6,7 g (0,1 Mol) Aethanolamin, 50 ml N,N-Dimethylformamid (DMF) und 30 ml Toluol wird gekocht, wobei das im Verlaufe der Reaktion entstehende Wasser azeotrop beseitigt wird. Das Reaktionsgemisch wird dann bis zur Trockne eingedampft. Der Rückstand wird in 200 ml Methylenchlorid gelöst und mit 100 ml gesättigter Natriumchloridlösung ausgeschüttelt. Die Methylenchloridlösung wird mit wasserfreiem Natriumsulfat getrocknet und eingedampft. Man erhält 18 g (76% d.Th.) N-(β-Hydroxyäthyl)-3-nitrophthalimid als öligen Rückstand, der in kurzer Zeit auskristallisiert; Fp. 93°C. IR—Spektrum ($CH_2Cl_2$): 1790 $cm^{-1}$ und 1730 $cm^{-1}$ (CO—N—CO); 1500 $cm^{-1}$ und 1370 $cm^{-1}$ ($CO_2$).

Beispiel 2

31,85 g (0,135 Mol) eines Gemisches von N-(β-Hydroxyäthyl)-3- und -4-nitrophthalsäureimid werden mit 9,62 g (0,148 Mol) Natriumazid in 360 ml Dimethylsulfoxid 18 Stunden auf 80°C erhitzt. Anschliessend wird die Lösung im Vakuum bei 80°C eingeengt und mit 250 ml Wasser verrührt. Die entstandene Suspension wird abgesaugt, in 100 ml Wasser wieder suspendiert und während 18 Stunden gerührt. Nach dem Absaugen und Trocknen der Suspension bei 70°C/100 Torr erhält man 24,5 g (78,6% d.Th.) eines Gemisches aus N-(β-Hydroxyäthyl)-3- und -4-azidophthalsäureimid; Fp. 102°C (Zersetzung). IR—Spektrum ($CH_2Cl_2$): 1785 $cm^{-1}$ und 1725 $cm^{-1}$ (CO—N—CO); 2125 $cm^{-1}$ ($N_3$).

Das N-(β-Hydroxyäthyl)-3- und -4-nitrophthalsäureimidgemisch kann wie folgt hergestellt werden: 38,6 g (0,2 Mol) eines Gemisches von 3- und 4-Nitrophthalsäureanhydrid, 13,4 g (0,22 Mol) Aethanolamin, 100 ml N,N-Dimethylformamid und 60 mi Toluol werden zum Rückfluss erhitzt, wobei das im Verlaufe der Reaktion entstehende Wasser azeotrop beseitigt wird. Das Reaktionsgemisch wird dann im Vakuum zur Trockne eingedampft. Der ölige Rückstand wird in 200 ml Methylenchlorid gelöst und zweimal mit gesättigter Natriumchloridlösung ausgeschüttelt. Die Methylenchloridlösung wird mit wasserfreiem Natriumsulfat getrocknet und bis zur Trockne eingedampft. Der ölige Rückstand kristallisiert durch Verrühren mit 500 ml Diäthyläther. Nach dem Absaugen und Trocknen der gebildeten gelben kristallinen Suspension bei 40°C/100 Torr erhält man 31,6 g (67% d.Th.) eines Gemisches aus N-(β-Hydroxyäthyl)-3- und -4-nitrophthalsäureimid; Fp. 80—95°C. IR—Spektrum (Dioxan): 1780 $cm^{-1}$ und 1720 $cm^{-1}$ (CO—N—CO); 1550 $cm^{-1}$ und 1340 $cm^{-1}$ ($NO_2$).

Beispiel 3

630 g (3,26 Mol) eines Isomerengemisches von 3- und 4-Nitrophthalsäureanhydrid werden in 1632 mol N,N-Dimethylformamid gelöst und mit 218 g (3,57 Mol) Aethanolamin und 980 ml Toluol versetzt. Das Reaktionsgemisch wird zum Rückfluss erhitzt, wobei das im Verlaufe der Reaktion entstehende Wasser azeotrop abdestilliert wird. Das Reaktionsgemisch wird dann im Vakuum bei 90°C

eingedampft. Der ölige Rückstand wird mit 7000 ml Dimethylsulfoxid und 263 g (4,04 Mol) Natriumazid versetzt. Anschliessend wird das Reaktionsgemisch 20 Stunden auf 80°C erhitzt, im Vakuum bei 90—100°C eingeengt und mit 5500 ml Wasser verrührt. Die entstandene gelbe Suspension wird abgesaugt und im Vakuum bei 60°C über festem Natriumhydroxid getrocknet. Man erhält 647 g (85,5% d.Th.) eines Isomerengemisches aus N-(β-Hydroxyäthyl)-3- und -4-nitrophthalsäureimid; Fp. 90—105°C (Zersetzung). IR—Spektrum ($CH_2Cl_2$): 1780 $cm^{-1}$ und 1720 $cm^{-1}$ (CO—N—CO); 2125 $cm^{-1}$ ($N_3$).

Beispiel 4

Ein Gemisch von 2,9 g (0,01 Mol) 4-(3-Nitrophthalimidyl)-cyclohexanol und 0,9 g (0,013 Mol) Natriumazid in 20 ml Dimethylsulfoxid wird 6 Stunden bei 80°C gerührt und nachher bei der gleichen Temperatur im Vakuum eingedampft. Der Rückstand wird mit 20 ml Wasser verdünnt. Das ausgeschiedene Produkt wird abgesaugt, mit 5 ml Wasser nachgewaschen und 24 Stunden bei 80°C im Trockenschrank getrocknet. Man erhält 2,6 g (91% d.Th.) 4-(3-Azidophthalimidyl)-cyclohexanol; Fp. 155°C (Zersetzung). IR—Spektrum (KBr): 1775 $cm^{-1}$ und 1710 $cm^{-1}$ (CO—N—CO); 2130 $cm^{-1}$ ($N_3$).

Das im obigen Beispiel verwendete Ausgangsprodukt kann wie folgt hergestellt werden: Ein Gemisch von 29,4 g (0,25 Mol) 4-Aminocyclohexanol, 43,9 g (0,277 Mol) 3-Nitrophthalsäureanhydrid, 120 ml N,N-Dimethylformamid und 75 ml Toluol wird unter azeotroper Beseitigung des während der Reaktion gebildeten Wassers zum Kochen erhitzt und schliesslich eingedampft. Der ölige Rückstand wird in 1000 ml Methylenchlorid gelöst und viermal mit jeweils 40,0 ml 5%iger wässriger NaOH—Lösung extrahiert. Die Methylenchloridlösung wird mit wasserfreiem Natriumsulfat getrocknet und eingedampft. Der ölige Rückstand wird mit 280 ml Methanol heiss gelöst. Beim Abkühlen kristallisieren 16 g (22% d.Th.) 4-(3-Nitrophthalimidyl)-cyclohexanol aus; Fp. 215°C. IR—Spektrum ($CH_2Cl_2$): 1790 $cm^{-1}$ und 1730 $cm^{-1}$ (CO—N—CO); 1550 $cm^{-1}$ und 1370 $cm^{-1}$ ($NO_2$).

Beispiel 5

Ein Gemisch von 55 g (0.193 Mol) 4-(3-Nitrophthalsäureimidyl)-phenol und 13,8 g (0,212 Mol) Natriumazid in 380 ml Dimethylsulfoxid wird 6 Stunden auf 50°C erhitzt. Das Reaktionsgemisch wird im Vakuum bei 80°C eingedampft, und der Rückstand wird mit 1000 ml Wasser während 18 Stunden gerührt. Nach dem Abfiltrieren und Trocknen der erhaltenen Suspension im Trockenschrank bei 60°C über Phosphorpentoxid erhält man 56,6 g (95% d.Th.) 4-(3-Azidophthalsäureimidyl)-phenol; Fp. 165°C(Zersetzung) IR—Spektrum (KBr): 1780 und 1715 $cm^{-1}$ (CO—N—CO); 2140 $cm^{-1}$ ($N_3$).

Das im obigen Beispiel verwendete Ausgangsprodukt kann wie folgt hergestellt werden: 34,3 g (0,31 Mol) 4-Aminophenol und 59,9 g (0,31 Mol) 3-Nitrophthalsäureanhydrid werden in 600 ml Essigsäure während 6 Stunden zum Rückfluss erhitzt und hierauf in 3000 ml Wasser eingerührt. Das ausgefallene Produkt wird mit Wasser gewaschen und 24 Stunden bei 70°C/30 Torr getrocknet. Man erhält 69 g (78% d.Th.) 4-(3-Nitrophthalsäureimidyl)-phenol; Fp. 202°C. IR—Spektrum (KBr): 1785 $cm^{-1}$ und 1725 $cm^{-1}$ (CO—N—CO); 1550 und 1350 $cm^{-1}$ ($NO_2$).

Beispiel 6

13,5 g (0,047 Mol) 4-(3-Nitrophthalsäureimidyl)-anilin und 3,4 g (0,051 Mol) Natriumazid in 120 ml Dimethylsulfoxid werden 18 Stunden bei 80°C erhitzt. Dann wird das Reaktionsgemisch im Vakuum bei 80°C eingeengt, mit 100 ml Wasser verdünnt, abgesaugt, mit 20 ml Wasser nachgewaschen und im Trockenschrank während 24 Stunden bei 80°C/100 Torr getrocknet. Man erhält 13,1 g (100% d.Th.) 4-(3-Azidophthalsäureimidyl)-anilin; Fp. 176°C (Zersetzung). IR—Spektrum (KBr): 1780 und 1725 $cm^{-1}$ (CO—N—CO); 2130 $cm^{-1}$ ($N_3$).

Das im obigen Beispiel verwendete 4-(3-Nitrophthalsäure)-anilin kann wie folgt hergestellt werden: 48,3 g (0,25 Mol) 3-Nitrophthalsäureanhydrid und 37,5 g (0,25 Mol) 4-Aminoacetanilid werden in 500 ml Essigsäure während 8 Stunden zum Rückfluss erhitzt und dann abgekühlt. Die ausgeschiedenen Kristalle werden abgesaugt, mit 150 ml Aethanol gewaschen und 24 Stunden bei 80°C/30 Torr getrocknet. Man erhält 65 g (80% d.Th.) 4-(3-Nitrophthalsäureimidyl)-acetanilid; Fp. 255°C. 65 g (0,2 Mol) 4-(3-Nitrophthalsäureimidyl)-acetanilid werden mit einem Gemisch von 400 ml Dioxan und 200 ml konzentrierter Salzsäure während 3 Stunden auf 90°C erhitzt und dann 24 Stunden bei 25°C stehen gelassen. Die ausgeschiedenen Kristalle werden abgesaugt und mit 100 ml Methanol gewaschen. Man erhält 45 g (70% d.Th.) rohes 4-(3-Nitrophthalsäureimidyl)-anilinhydrochlorid. 45 g (0,14 Mol) dieses Hydrochlorids werden mit 100 ml Wasser vermischt, und bei 5°C werden 200 ml einer gesättigten Natriumbicarbonatlösung zugetropft. Nach einer Stunde wird der entstandene feine Niederschlag abgesaugt, dreimal mit 50 ml Wasser gewaschen und 24 Stunden bei 25°C/1 Torr getrocknet. Man erhält 21 g (54% d.Th.) 4-(3-Nitrophthalsäureimidyl)-anilin; Fp. 177°C. IR—Spektrum (KBr): 1790 und 1725 $cm^{-1}$ (CO—N—CO); 1540 und 1360 $cm^{-1}$ ($NO_2$).

Beispiel 7

Ein Gemisch von 3,12 g (0,01 Mol) 4-(3-Nitrophthalsäureimidyl)-benzoesäure in 20 ml Dimethylsulfoxid wird zuerst mit 1,4 ml (0,01 Mol) Triäthylamin und dann mit 0,72 g (0,011 Mol) Natriumazid versetzt. Das Reaktionsgemisch wird 6 Stunden auf 50°C erhitzt, im Vakuum bei 80°C eingedampft

und mit 70 ml Wasser verrührt, worauf man die entstande Lösung mit Salzsäure auf pH 1 stellt. Das ausgefallene Produkt wird abgesaugt, mit 5 ml Methanol nachgewaschen und im Trockenschrank bei 80°C/100 Torr 24 Stunden getrocknet. Man erhält 2,47 g (80% d.Th.) 4-(3-Azidophthalsäureimidyl)-benzosäure; Fp. 300°C (Zersetzung). IR—Spektrum (KBr): 1790 und 1740 $cm^{-1}$ (CO—N—CO); 2150 $cm^{-1}$ ($N_3$).

Das im obigen Beispiel verwendete Ausgangsprodukt kann wie folgt hergestellt werden: 57,9 g (0,3 Mol) 3-Nitrophthalsäureanhydrid und 41,1 g (0,3 Mol) 4-Aminobenzoesäure werden in 600 ml Essigsäure 6 Stunden zum Rückfluss erhitzt und nachher in 1000 ml 50%igen wässrigen Aethanol eingerührt. Das ausgefallene gelbe Produkt wird abgesaugt, mit Wasser gewaschen und bei 80°C/30 Torr 24 Stunden getrocknet. Man erhält 82 g (88% d.Th.) 4-(3-Nitrophthalsäureimidyl)-benzoesäure; Fp. > 300°C. IR—Spektrum (KBr): 1790 und 1740 $cm^{-1}$ (CO—N—CO); 1550 und 1370 $cm^{-1}$ ($NO_2$).

Beispiel 8

Ein Gemisch von 5,7 g (0.018 Mol) 6-(3-Nitrophthalsäureimidyl)-capronsäure in 35 ml Dimethylsulfoxid wird zuerst mit 2,58 ml (0,018 Mol) Triäthylamin und dann mit 1,3 g (0,0198 Mol) Natriumazid versetzt. Das Reaktionsgemisch wird 6 Stunden auf 50°C erhitzt, im Vakuum eingeengt, mit 100 ml Wasser verdünnt und mit 4 ml konz. Salzsäure angesäuert. Die entstandene Suspension des Reaktionsproduktes wird 18 Stunden gerührt, abgesaugt, mit 20 ml Wasser nachgewaschen und 24 Stunden über Phosphorpentoxid bei 60°C/Torr getrocknet. Man erhält 5,1 g (94% d.Th.) 6-(3-Azidophthalsäureimidyl)-capronsäure; Fp. 87—90°C (Zersetzung). IR—Spektrum ($CH_2Cl_2$): 1780 und 1725 $cm^{-1}$ (CO—N—CO); 2130 $cm^{-1}$ ($N_3$).

Das im obigen Beispiel verwendete Ausgangsprodukt kann wie folgt hergestellt werden: 3,86 g (0,02 Mol) 3-Nitrophthalsäureanhydrid und 2,9 g (0,022 Mol) 6-Aminocapronsäure werden 8 Stunden am Rückfluss erhitzt und darauf eingedampft. Der feste Rückstand wird mit Wasser verrührt, abgesaugt und bei 80°C während 24 Stunden getrocknet. Man erhält 5,7 g (93% d.Th.) 6-(3-Nitrophthalsäureimidyl)-capronsäure; Fp. 148—150°C. IR—Spektrum ($CH_2Cl_2$): 1790 und 1725 $cm^{-1}$ (CO—N—CO); 1550 und 1370 $cm^{-1}$ ($NO_2$).

Beispiel 9

Die Lösungen von 3,78 g (0,02 Mol) 3-Azidophthalsäureanhydrid in 50 ml Dioxan und 2,74 g (0,02 Mol) p-Aminobenzoesäure in 50 ml Dioxan werden vermischt und 3 Stunden stehen gelassen. Danach wird 5 ml Acetanhydrid zugegeben, und das Reaktionsgemisch wird 1 Stunde auf 85°C erhitzt. Nach dem Einengen bis zur Trockne wird der Rückstand mit Wasser vermischt, und die entstandenen Kristalle werden abgesaugt. Man erhält 4,8 g (78% d.Th.) 4-(3-Azidophthalsäureimidyl)-benzoesäure; Fp. 300°C (Zersetzung).

Beispiel 10

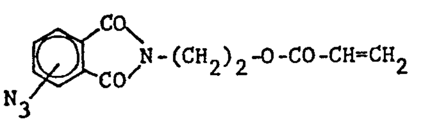

In einem 500 ml Sulfierkolben, der mit einem 50 ml-Tropftrichter mit Druckausgleich, einem Intensivkühler mit Trokkenrohr und einem Thermometer ausgerüstet ist, werden unter Stickstoffatmosphäre 23,1 g (0,1 Mol) des gemäss Beispiel 2 erhaltenen Gemisches von N-($\beta$-Hydroxyäthyl)-3- und -4-azidophthalsäureimid und 10,1 g (0,1 Mol) Triäthylamin (über NaOH getrocknet) in 250 ml trockenem Dichlormethan gelöst und auf 0°C abgekühlt. Zu dieser Lösung werden 9,05 g (0,1 Mol) Acrylsäurechlorid so zugetropft, dass die Temperatur 10°C nicht übersteigt. Nach beendeter Reaktion wird so lange ausgerührt, bis sich das Reaktionsgemisch auf Raumtemperatur (20—25°C) erwärmt hat. Das bei der Reaktion ausgefallene Triäthylaminhydrochlorid wird nun durch Filtration von der übrigen Reaktionslösung getrennt. Der Dichlormethanextrakt wird mit Wasser neutral gewaschen, mit Na-Sulfat getrocknet und anschliessend am Vakuum ohne zu heizen eingeengt. Man erhält 25,9 g (90,7% d.Th.) eines Gemisches von N-($\beta$-Acryioyloxyäthyl)-3- und -4-azidophthalsäureimid. NMR—Spektrum: $H_2C{=}CH$— 5,7—6,3 (3H) ppm; (interner Standard TMS = 0).

Beispiel 11

In einer Apparatur der im Beispiel 10 beschriebenen Art werden unter Stickstoffatmosphäre 23,2 g (0,1 Mol) eines Gemisches von N-($\beta$-Hydroxyäthyl)-3- und -4-azidophthalsäureimid und 10,1 g (0,1 Mol) Triäthylamin (über NaOH getrocknet) in 250 ml trockenem Dichlormethan gelöst und auf 0°C gekühlt. Zu dieser Lösung werden 10,45 g (0,1 Mol) Methacrylsäurechlorid so zugetropft, dass die Temperatur 10°C nicht übersteigt. Nach beendeter Reaktion wird so lange gerührt, bis sich das Reaktionsgemisch auf Raumtemperatur erwärmt hat. Das bei der Reaktion ausgefallene Triäthylaminhydrochlorid wird nun durch Filtration von der übrigen Reaktionslösung getrennt. Der Dichlormethanextrakt wird mit Wasser neutral gewaschen, mit Na-Sulfat getrocknet und am Vakuum

ohne zu heizen eingeengt. Man erhält 27,65 g (92,2% d.Th.) eines Gemisches von N-(β-Methacryloyloxyäthyl)-3- und -4-azidophthalsäureimid. NMR—Spektrum:

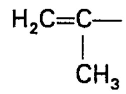

6,1 (1H) und 5,6 (1H) ppm; interner Standard TMS = 0.

Beispiel 12

Unter Gelblicht werden 15,0 g (0,05 Mol) der gemäss Beispiel 8 erhaltenen 6-(3-Azidophthalsäureimidyl)-capronsäure in 327 g (2,75 Mol) Thionylchlorid gelöst und auf 80°C erwärmt. Bei dieser Temperatur gibt man zur entstandenen klaren Lösung 0,5 ml N,N-Dimethylformamid. Das Reaktionsgemisch wird während 15 Minuten bei 80°C gerührt und dann auf Raumtemperatur abgekühlt. Dabei entsteht eine beige-farbene Suspension, welche unter $N_2$ genutscht wird. Das Rohprodukt wird aus 250 ml trockenem Ligroin umkristallisiert. Man erhält 12,5 g (75,4% d.Th.) 6-(3-Azidophthalsäureimidyl)-capronsäurechlorid; Fp. 68—69°C.

Elementaranalyse:

| | | | | |
|---|---|---|---|---|
| berechnet | C 52,43% | H 4,09% | N 17,47% | Cl 11,06% |
| gefunden | C 52,61% | H 4,13% | N 17,27% | Cl 9,04% |

Beispiel 13

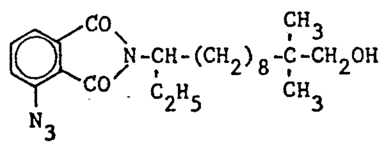

Ein Gemisch von 20,9 g (0,05 Mol) N-(1-Aethyl-10,10-dimethyl-11-hydroxyundecyl)-3-nitrophthalsäureimid und 3,55 g (0,055 Mol) Natriumazid in 100 ml Dimethylsulfoxid wird 24 Stunden bei 80°C gerührt. Die Lösung wird im Vakuum eingedampft und mit 300 ml Wasser verdünnt. Die entstandene Emulsion wird zweimal mit 100 ml Diäthyläther extrahiert. Die Aetherextrakte werden zuerst mit wasserfreiem Natriumsulfat getrocknet und dann eingedampft. Man erhält 14,7 g (71% d.Th.) N-(1-Aethyl-10,10-dimethyl-11-hydroxyundecyl)-3-azidophthalsäureimid in Form eines zähen, nicht destillierbaren Oels.

IR-Spektrum ($CHCl_3$): 1780 und 1725 $cm^{-1}$ (CO—N—CO); 2130 $cm^{-1}$ ($N_3$).

Das im obigen Beispiel verwendete Ausgangsprodukt kann wie folgt hergestellt werden: Ein Gemisch von 9,6 g (0,05 Mol) 3-Nitrophthalsäureanhydrid, 12,9 g (0,055 Mol) 1-Aethyl-10,10-dimethyl-11-hydroxy-1-aminoundecan, 150 ml Toluol und 250 ml Dimethylformamid wird zum Kochen erhitzt, wobei das entstehende Wasser azeotrop beseitigt wird. Das Reaktionsgemisch wird dann bis zur Trockne eingedampft. Der Rückstand wird in 150 ml Methylenchlorid gelöst und dreimal mit je 50 ml 10%iger Sodalösung extrahiert. Die Methylenchloridlösung wird dann über wasserfreiem Natriumsulfat getrocknet und eingedampft. Man erhält 20,9 g (ca. 100% d.Th.) N-(1-Aethyl-10,10-dimethyl-11-hydroxyundecyl)-3-nitrophthalsäureimid in Form eines zähen Oels, das nach mehrwöchigem Stehenlassen allmählich kristallisiert; Fp. 66—68°C.

Beispiel 14

Ein Gemisch von 2,4 g (0,007 Mol) 1-(3-Nitrophthalsäureimidyl)-7-hydroxynaphthalin und 0,52 g (0,008 Mol) Natriumazid in 14 ml Dimethylsulfoxid wird bei 80°C 24 Stunden gerührt. Die Lösung wird mit 100 ml Wasser versetzt. Die ausgefallenen Kristalle werden abgesaugt, mit Wasser gewaschen und bei 80°C/100 Torr getrocknet. Man erhält 1,7 g (73,6% d.Th.) 1-(3-Azidophthalsäureimidyl)-7-hydroxynaphthalin; Fp. 154°C (Zersetzung). IR—Spektrum (KBr): 1775 und 1720 $cm^{-1}$ (CO—N—CO); 2130 $cm^{-1}$ ($N_3$).

Das im obigen Beispiel verwendete Ausgangsprodukt kann wie folgt hergestellt werden: Ein Gemisch von 1,93 g (0,01 Mol) 3-Nitrophthalsäureanhydrid, 1,59 g (0,01 Mol) 1-Amino-7-naphthol und 30 ml Essigsäure werden 17 Stunden zum Rückfluss erhitzt. Die entstandene Suspension wird mit 300 ml Wasser verrührt, dann mit Wasser nachgewaschen und getrocknet. Man erhält 2,9 g Rohprodukt, das mit Chloroform extrahiert wird. Der Chloroformextrakt wird mit 4 g Kieselgel verrührt, heiss abgesaugt und bis zur Trockne eingedampft. Man erhält 2,4 g (72% d.Th.) 1-(3-Nitrophthalsäureimidyl)-7-hydroxynaphthalin; Fp. 136—7°C.

Beispiel 15

In einem 1-Liter Reaktionsgefäss, das mit Doppelmantel, Rührer, Intensivkühler und Thermometer ausgestattet ist, werden unter Gelblicht 120,0 g eines Gemisches aus N-(β-Methacryloyloxyäthyl)-3-

und -4-azidophthalimid und 1,20 g Azoisobutyronitril in 545 ml Tetrahydrofuran gelöst. Diese Lösung wird unter Umrühren und unter Stickstoffatmosphäre während 8 Stunden bei 70°C polymerisiert. Nach beendeter Reaktion wird das Reaktionsgemisch auf Raumtemperatur (20—25°C) abgekühlt, und das Polymere wird durch Eintropfen der Reaktionslösung in 4 Liter n-Hexan ausgefällt. Man erhält 113,8 g (94,8% d.Th.) eines schwach gelblichen Polymeren; inhärente Viskosität $\eta = 0,22$ dl/g (c = 0,5 Gew.% in N,N-Dimethylformamid bei 25°C).

Zur Herstellung von photoempfindlichen Platten, z.B. zur Herstellung von gedruckten Schaltungen wird nach an sich bekannter Herstellungstechnik [vgl. Bogenschütz in "Fotolacktechnik", Eugen G.Lenze-Verlag, DT 7968 Saulgau (1975)] mit einer 5%igen Lösung des obigen Polymeren in N,N-Dimethylformamid (DMF) eine kupferkaschierte Epoxiplatte so beschichtet, dass nach dem Trocknen bei 40°C eine ca. 5 $\mu$ dicker Film gebildet wird. Belichtet man nun diese Platte durch ein Strichnegativ mit UV Lickt ($\lambda$ über 320 nm) während einer Minute, so erhält man nach dem Entwickeln der unvernetzten Anteile in Tetrahydrofuran und dem Wegätzen der ungeschützten Kupferoberfläche die dem Strichnegativ entsprechende Schaltung.

Beispiel 16

In einer Apparatur der in Beispiel 15 beschriebenen Art werden unter Gelblicht 105,0 g eines Gemisches von N-($\beta$-Methacryloyloxyäthyl)-3- und -4-azidophthalimid, 15,0 g Acrylsäureäthylester und 1,20 g Azoisobutyronitril in 545 ml Tetrahydrofuran gelöst. Diese Lösung wird unter Umrühren und unter Stickstoffatmosphäre während 8 Stunden bei 70°C polymerisiert. Nach beendeter Reaktion wird auf Raumtemperatur abgekühlt, und das erhaltene Polymere wird durch Eintropfen der Reaktionslösung in 4 Liter n-Hexan ausgefällt. Man erhält 113,8 g (94,8% d.Th.) eines schwach gelblichen Polymeren; inhärente Viskosität $\eta = 0,28$ dl/g (c = 0,5 Gew.% in DMF bei 25°C).

Beispiel 17

In einem 250 ml Rundkolben, der mit Intensivkühler und Trockenrohr ausgestattet ist, werden 2,5 g N-($\beta$-Hydroxyäthyl)-3-azidophthalimid, 5,0 g eines Copolymeren aus Methylvinyläther und Maleinsäureanhydrid (GANTREZ 119; Anhydridgehalt 0,64 Mol; $\eta = 5,76$ cP; Handelsprodukt der Fa. General Aniline + Film Corp.), d.h. ein Polymeres mit wiederkehrenden Strukturelementen der Formel

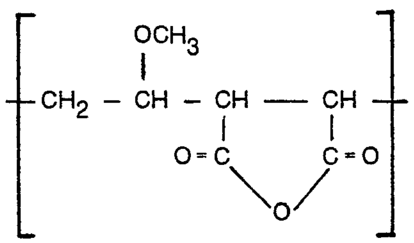

und 0,1 ml konzentrierte Schwefelsäure in 77 ml Tetrahydrofuran gelöst. Nach dem Aufheizen des Reaktionsgemisches wird dieses unter Umrühren während 48 Stunden am Rückfluss gehalten. Nach dem Erkalten der Reaktionslösung wird das erhaltene Polymere durch Eintropfen der Reaktionslösung in 500 ml Diäthyläther ausgefällt. Man erhält 6,8 g (91% d.Th.) eines hellgelben Polymeren, das 33,5 Gew.% Azid enthält.

Beispiel 18

In einer Apparatur der in Beispiel 17 beschriebenen Art werden 2,5 g N-($\omega$-Carboxypentyl)-3-azidophthalimid, 2,5 g eines Styrol/Methacrylsäure-glycidylester-Copolymerisats (Molverhältnis 1:1, Molekulargewicht 25'000, d.h. eine Copolymerisat mit wiederkehrenden Strukturelementen der Formel

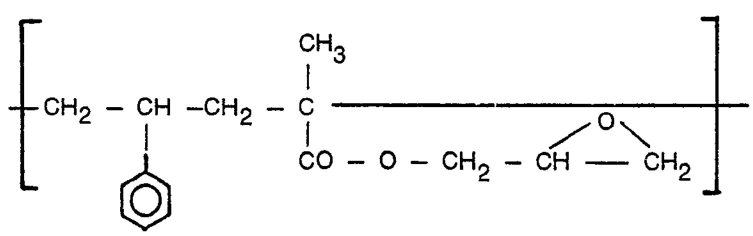

und 0,03 g Tetramethylammoniumchlorid in 45 ml Cyclohexanon gelöst. Nach dem Aufheizen des Reaktionsgemisches wird während 1 3/4 Stunden bei 110°C gerührt. nach dem Abkühlen wird das Polymere durch Eintropfen der Reaktionslösung in 300 ml n-Hexan ausgefällt. Man erhält 4,49 g (89,9% d.Th.) eines hellgelben Polymeren, das 50 Gew.% Azid enthält.

Beispiel 19

In einer Apparatur der in Beispiel 17 beschriebenen Art werden 0,7 g N-(4-Carboxyphenyl)-3-azidophthalimid, 1,4 g eines Methacrylsäuremethylester/Methacrylsäure-glycidylester-Copolymeren mit wiederkehrenden Strukurelementen der Formel

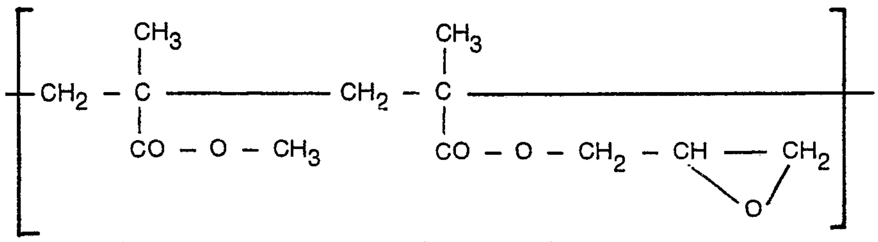

(Molverhältnis 1:1, Molekulargewicht 25000) und 0,005 g Tetramethylammoniumchlorid in 25 ml Cyclohexanon gelöst. Nach dem Aufheizen wird das Reaktionsgemisch während 2 Stunden bei 110°C gerührt. Durch Eintropfen der Reaktionslösung nach dem Abkühlen in 150 ml n-Hexan wird das Polymere ausgefällt. Man erhält 1,96 g (93,3% d.Th.) eines hellgelben Polymeren, das 33,5 Gew.% Azid enthält.

Beispiel 20

Auf analoge Weise wie in den Beispielen 15 und 16 beschrieben wird ein Gemisch von N-($\beta$-Methacryloyloxyäthyl)-3- und -4-azidophthalimid mit Acrylsäure in einem Gewichtsverhältnis von 1:4 während 20 Stunden bei 60°C in Gegenwart von 0,5 Gew.% Azoisobutyronitril, bezogen auf das Gewicht der Monomeren, polymerisiert. Das erhaltene Polymere wird durch Eintropfen der Reaktionslösung in Diäthyläther ausgefällt. Man erhält ein schwach gelbliches Polymeres; inhärente Viskosität $\eta = 0{,}23$ dl/g (c = 0,5 Gew.% in DMF bei 25°C).

Die gemäss den Beispielen 16—20 erhaltenen Polymeren können auf die beschriebene Weise zum Herstellen von gedruckten Schaltungen verwendet werden.

Beispiel 21

Das gemäss Beispiel 20 erhaltene Polymere wird in einem photographischen Schichtverband geprüft. Zurest wird eine Giesslösung folgender Zusammensetzung zubereitet:

| | |
|---|---|
| Gelatine | 2 g/m$^2$ |
| Polymeres gemäss Beispiel 20 | 1 g/m$^2$ |
| Benetzungsmittel (Polyäthylenoxid-Stearat) | 20 Gew.%, bezogen auf das Gewicht des Polymeren |
| Photosensibilisator (2-p-Methoxybenzyl-6'- und -7'-sulfochinoxalin) | 4 Gew.%, bezogen auf das Gewicht des Polymeren |
| Härter (2-Hydroxy-6-amino-s-triazin-4-N-methylmorpholinium-Tetrafluorborat) | 8 Gew.%, bezogen auf das Gewicht der Gelatine. |

Diese Giesslösung wird auf einen transparenten Träger, d.h. auf eine Polyester-Folie, gegossen. Nach dem Aushärten der Gelatine wird der Film durch ein Raster-Negativ (Stufenkeil mit 12 Stufen) während 15 Sekunden mit einer 400 Watt Hochdrucklampe belichtet. Die nicht belichteten Anteile werden während 20 Sekunden in 20°C warmem Wasser ausgewaschen. Das vernetzte Polymere wird mit einem kationischen Farbstoff, z.B. dem Farbstoff der Formel

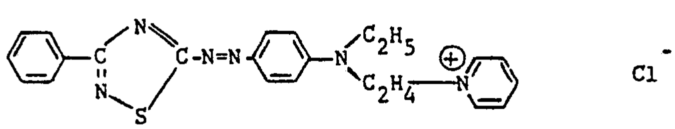

aus wässriger Lösung angefärbt bzw. sichtbar gemacht, wobei sämtliche 12 Stufen des Stufenkeils wiedergegeben werden.

Beispiel 22

Unter Gelblicht werden 10,42 g (0,035 Mol) N-($\beta$-Methacryloyloxyäthyl)-3-azidophthalimid und 2,32 g (0,023 Mol) Acrylsäureäthylester in 50 ml Tetrahydrofuran gelöst und unter Stickstoff auf 70°C

erwärmt. In 7 ml Tetrahydrofuran werden 0,127 g Azo-isobutyronitril gelöst und über einen mit Stickstoff gespülten Tropftrichter in die Monomerlösung eingetragen. Das Reaktionsgemisch wird während 7 Stunden bei 70°C ausgerührt, dann auf Raumtemperatur gekühlt, filtriert und auf 1 Liter Diäthyläther ausgefällt. Die leicht gelbliche Suspension wird abgenutscht, und das erhaltene Produkt wird im Vakuum bei 30°C getrocknet. Ausbeute: 11,85 g = 93% d.Th.; 11,8 Gew.% N; inhärente Viskosität $\eta$ 0,18 dl/g (c = 0,5 Gew.% in DMF bei 25°C).

Eine kupferbeschichtete Platte (ca. 5 × 10 cm) wird auf einer handelsüblichen Zentrifuge mit einer 10%igen Lösung des obigen Polymeren in DMF (Viskosität ca. 1 Pa s) beschichtet. Die so mit dem Photolack versehene Kupferplatte wird anschliessend bei 60°C in einem Umluftofen getrocknet. Die beschichtete Platte wird durch eine 21-Stufen-Filmnegativ-Vorlage (sog. "21-step sensitivity guide") mit einer 400 Watt Quecksilberhochdrucklampe mit vorgeschaltetem Pyrex-Glasfilter während verschieden langen Zeitintervallen im Abstand von 60 cm belichtet. Die belichtete Platte wird in 1,1,1-Trichloräthan entwickelt und in $FeCl_3$-Lösung angeätzt. Zur Ermittlung der Lichtempfindlichkeit wird jeweils die letzte sichtbare Stufe der Stufenvorlage nach dem Anätzen angegeben:

| Belichtungszeit | Stufe |
|---|---|
| 1 Minute | 0 |
| 3 Minuten | 0—1 |
| 6 Minuten | 4 |

*Vergleichsbeispiel:* Auf die im Beispiel 15 beschriebene Weise wird N-($\beta$-Hydroxyäthyl)-3-azido-phthalimid mit einem Maleinsäureanhydrid-Polymeren ("Gantrez AN", Handelsprodukt der Fa. GAF) zu einem Polymeren mit wiederkehrenden Strukturelementen der Formel

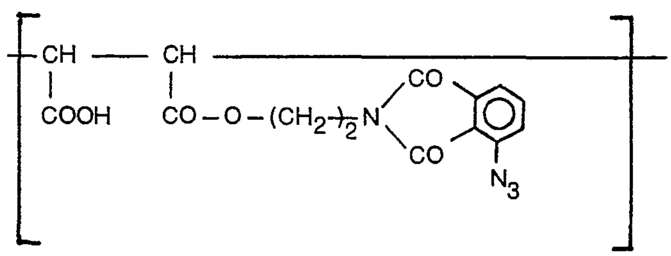

(A) umgesetzt.

Dasselbe Maleinsäureanhydrid-Polymere wird auf analoge Weise mit 3-Azidophthalsäuremono-$\beta$-hydroxyäthylester zu einem Polymeren mit wiederkehrenden Strukturelementen der Formel

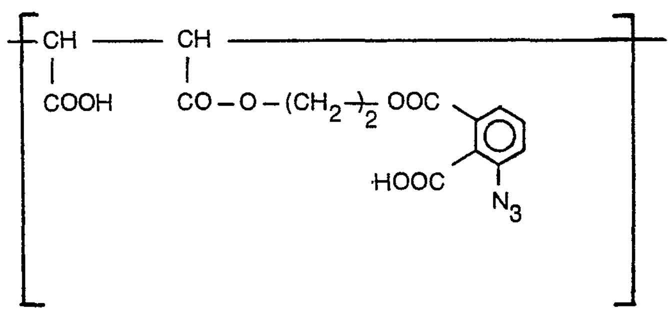

(B) umgesetzt.

Bestrahlt man die Polymeren (A) und (B) mit UV—Licht von über 320 nm, so zeigt das Polymere (A) gegenüber dem Polymeren (B) eine 5—10mal höhere Lichtempfindlichkeit.

**Patentansprüche**

1. 3-oder 4-Azidophthalsäurederivate der Formeln Ia oder Ib

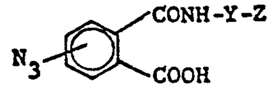

(Ia)

oder

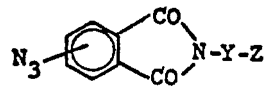

(Ib), worin

Y unsubstituiertes oder substituiertes Alkylen mit 2—18 C—Atomen, eine unsubstituierte oder

substituierte Phenylen-, Naphthylen-, Biphenylen-, Cyclohexylen- oder Dicyclohexylmethangruppe oder eine unsubstituierte oder substituierte Gruppierung

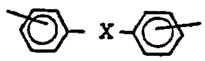

X —O—, —S—, $—SO_2—$, $—CH_2—$, $—\underset{\displaystyle CH_3}{\underset{|}{CH}}—$, $—\underset{\displaystyle CH_3}{\underset{|}{\overset{\displaystyle CH_3}{\overset{|}{C}}}}—$ oder —CO— und

Z $—NH_2$, —NH—Alkyl mit 1—4 C—Atomen, —OH, —COOH, —COCl, $—O—CO—CH{=}CH_2$, $—O—CO—\underset{\displaystyle CH_3}{\underset{|}{C}}{=}CH_2$, $—COO—CH{=}CH_2$ oder $—O—CH{=}CH_2$ darstellen.

2. Eine Verbindung der Formel Ia oder Ib nach Anspruch 1, worin Y eine unsubstituierte oder durch eine oder zwei Phenylgruppen, Cycloalkylgruppen mit 5—8 C—Atomen oder Aralkylgruppen mit 7 oder 8 C—Atomen substituierte Alkylengruppe mit 2—18 C—Atomen oder unsubstituiertes oder pro Ring durch ein Halogenatom, eine Alkylgruppe mit 1—4 C—Atomen, eine Cycloalkylgruppe mit 5—7 C—Atomen oder eine Aralkylgruppe mit 7 oder 8 C—Atomen substituiertes Phenylen, Naphthylen, Biphenylen, Cyclohexylen, Dicyclohexylmethan oder

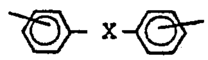

darstellt und Z und X die unter Formel Ia bzw. Ib angegebene Bedeutung haben.

3. Eine Verbindung der Formel Ia oder Ib nach Anspruch 1, worin die $N_3$-Gruppe in ortho-Stellung zur Carboxyl- oder Carbonamidgruppe bzw. zu einer Carbonylgruppe an den Benzolring gebunden ist.

4. Eine Verbindung der Formel Ia oder Ib nach Anspruch 1, worin Y unsubstituiertes Alkylen mit 2—18, besonders 2—16 C—Atomen, Cyclohexylen, Naphthylen oder Phenylen und Z $—NH_2$, —COOH, —COCl, —OH, $—O—CO—CH{=}CH_2$ oder $—O—CO—\underset{\displaystyle CH_3}{\underset{|}{C}}{=}CH_2$ bedeuten.

5. Eine Verbindung nach Anspruch 1 der Formel VII

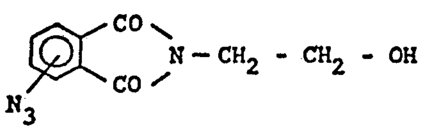

(VII),

vorzugsweise das N-(β-Hydroxyäthyl)-3-azidophthalimid.

6. Eine Verbindung nach Anspruch 1 der Formel VIII

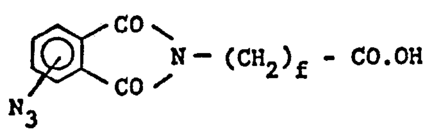

(VIII),

worin f eine Zahl von 2 bis 5, vorzugsweise 5 bedeutet, vorzugsweise die 6-(3-Azidophthalsäureimidyl)-capronsäure.

7. Eine Verbindung nach Anspruch 1 der Formel IX

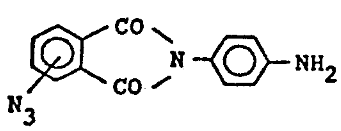

(IX),

vorzugsweise das 4-(3-Azidophthalsäureimidyl)-anilin.

8. Eine Verbindung nach Anspruch 1 der Formel X

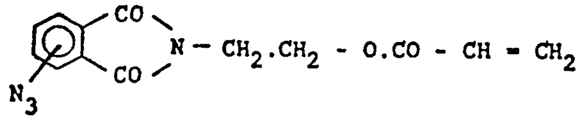

(X).

9. Eine Verbindung nach Anspruch 1 der Formel XI

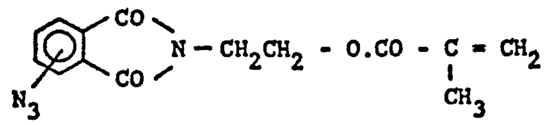 (XI).

10. Ein Verfahren zur Herstellung einer Verbindung der Formel Ib nach Anspruch 1, dadurch gekennzeichnet, dass man eine Verbindung der Formel II

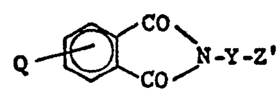 (II),

worin Y die unter Formel Ib angegebene Bedeutung hat, Z' eine Z entsprechende Gruppierung darstellt, aber ungleich —COCl ist und Q ein Halogenatom oder die Nitrogruppe bedeutet, in einem inerten organischen Lösungsmittel bei einer Temperatur zwischen etwa 0°C und 120°C mit einem Azid der Formel III

$$M^{+n}\,(N_3^-)_n \qquad \text{(III)}$$

umsetzt, wobei n die Zahl 1 oder 2 und M ein Alkalimetall-, Erdalkalimetall- oder quaternäres Ammoniumkation bedeuten, und die erhaltene Verbindung der Formel Ib, worin Z —COOH darstellt, gegebenenfalls durch Behandeln mit einem Chlorierungsmittel in das entsprechende Säurechlorid überführt.

11. Ein Verfahren nach Anspruch 10, dadurch gekennzeichnet, dass man eine Verbindung der Formel II verwendet, worin Q die Nitrogruppe darstellt.

12. Ein Verfahren nach Anspruch 10, dadurch gekennzeichnet, dass man als Azid der Formel III ein Alkalimetallazid, besonders Natriumazid, verwendet.

13. Ein Verfahren nach Anspruch 10, dadurch gekennzeichnet, dass man die Umsetzung bei einer Temperatur zwischen etwa 50 und 90°C vornimmt.

14. Ein Verfahren zur Herstellung einer Verbindung der Formel Ia oder Ib nach Anspruch 1, worin Z ungleich $—OCH{=}CH_2$ ist, dadurch gekennzeichnet, dass man eine Verbindung der Formel IV

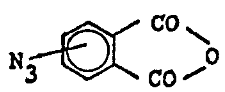 (IV)

mit einem Amin der Formel V $H_2N—Y—Z''$ (V)

zu einer Verbindung der Formel VI

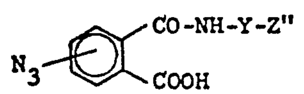 (VI)

umsetzt, wobei Y die unter Formel Ia bzw. Ib angegebene Bedeutung hat und Z'' $—NH_2$, —NH—Alkyl mit 1—4 C—Atomen, —OH, —COOH, $—O—CO—CH{=}CH_2$, $—O—CO—\underset{CH_3}{\underset{|}{C}}{=}CH_2$ oder $—COO—CH{=}CH_3$ darstellt, die Verbindung der Formel VI gegebenenfalls anschliessend cyclisiert und eine so erhaltene Verbindung der Formel Ib, worin Z —COOH darstellt, gewünschtenfalls durch Behandeln mit einem Chlorierungsmittel in das entsprechende Säurechlorid überführt.

**Claims**

1. 3- or 4-Azidophthalic acid derivatives

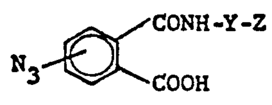 or 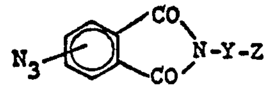

(Ia) (Ib)

in which Y is unsubstituted or substituted alkylene having 2—18 C atoms, an unsubstituted or substituted phenylene, naphthylene, biphenylene, cyclohexylene or dicyclohexylmethane group or an unsubstituted or substituted

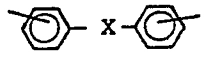

group, X is —O—, —S—, —$SO_2$—, —$CH_2$—, $-\underset{CH_3}{\underset{|}{CH}}-$, $-\overset{CH_3}{\overset{|}{\underset{CH_3}{\underset{|}{C}}}}-$ or —CO— and Z is —$NH_2$, —NH-alkyl having 1—4 C atoms, —OH, —COOH, —COCl, —O—CO—CH=$CH_2$, $-O-CO-\underset{CH_3}{\underset{|}{C}}=CH_2$, —COO—CH=$CH_2$ or —O—CH=$CH_2$.

2. A compound of the formula Ia or Ib according to claim 1, in which Y is an alkylene group, having 2—18 C atoms, which is unsubstituted or is substituted by one or two phenyl groups, cycloalkyl groups having 5—8 C atoms or aralkyl groups having 7 or 8 C atoms, or is phenylene, naphthylene, biphenylene, cyclohexylene, dicyclohexylmethane or

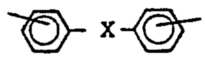

which are unsubstituted or are substituted, per ring, by one halogen atom, one alkyl group having 1—4 C atoms, one cycloalkyl group having 5—7 C atoms or one aralkyl group having 7 or 8 C atoms, and Z and X are as defined under formula Ia or Ib.

3. A compound of the formula Ia or Ib according to claim 1, in which the $N_3$ group is joined to the benzene ring in the ortho-position to the carboxyl or carboxamide group, or to a carbonyl group.

4. A compound of the formula Ia or Ib according to claim 1, in which Y is unsubstituted alkylene having 2—18, especially 2—16, C atoms, cyclohexylene, naphthylene or phenylene and Z is —$NH_2$, —COOH, —COCl, —OH, —O—CO—CH=$CH_2$ or $-O-CO-\underset{CH_3}{\underset{|}{C}}=CH_2$.

5. A compound according to claim 1, of the formula VII

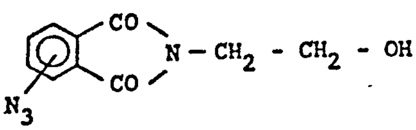 (VII),

preferably N-(β-hydroxyethyl)-3-azidophthalimide.

6. A compound according to claim 1, of the formula VIII

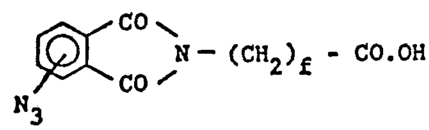 (VIII),

in which f is from 2 to 5, preferably 5, preferably 6-(3-azidophthalimidyl)-caproic acid.

7. A compound according to claim 1, of the formula IX

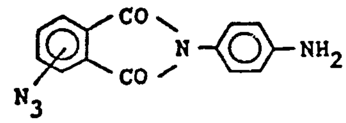 (IX),

preferably 4-(3-azidophthalimidyl)-aniline.

8. A compound according to claim 1, of the formula X

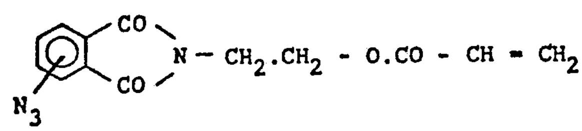 (X).

9. A compound according to claim 1, of the formula XI

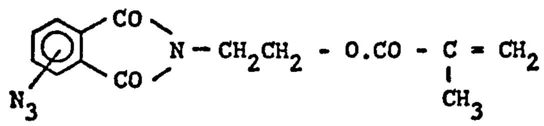

(XI).

10. A process for the preparation of a compound of the formula Ib according to claim 1, which comprises reacting a compound of the formula II

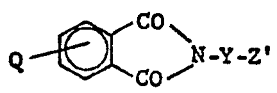

(II)

in which Y is defined as under formula Ib, Z′ is a group corresponding to Z but is not —COCl and Q is a halogen atom, or is the nitro group, in an inert organic solvent, at a temperature between about 0°C and 120°C, with an azide of the formula III

$$M^{n+} (N_3^-)_n \qquad (III)$$

in which n is 1 or 2 and M is an alkali metal cation, alkaline earth metal cation or quaternary ammonium cation, and if desired converting the resulting compound of the formula Ib, in which Z is —COOH, to the corresponding acid chloride by treatment with a chlorinating agent.

11. A process according to claim 10, wherein a compound of the formula II, in which Q is the nitro group, is used.

12. A process according to claim 10, wherein an alkali metal azide, especially sodium azide, is used as the azide of the formula III.

13. A process according to claim 10, wherein the reaction is carried out at a temperature between about 50 and 90°C.

14. A process for the preparation of a compound of the formula Ia or Ib according to claim 1, in which Z is not $—OCH=CH_2$, which process comprises reacting a compound of the formula IV

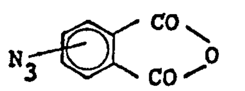

(IV)

with an amine of the formula V $\quad H_2N—Y—Z'' \quad$ (V)

to give a compound of the formula VI

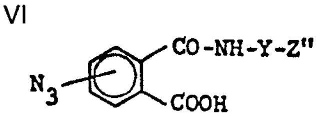

(VI),

in which formulae Y is as defined under formula Ia or Ib and Z″ is $—NH_2$, —NH-alkyl having 1—4 C atoms, —OH, —COOH, $—O—CO—CH=CH_2$, $—O—CO—C(CH_3)=CH_2$ or $—COO—CH=CH_2$, thereafter, if appropriate, cyclising the compound of the formula VI and, if desired, converting a resulting compound of the formula Ib, in which Z is —COOH, to the corresponding acid chloride by treatment with a chlorinating agent.

**Revendications**

1. Dérivés de l'acide azido-3 ou azido-4 phtalique qui répondent à l'une des formules (Ia) et (Ib)

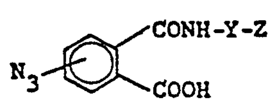

(Ia)

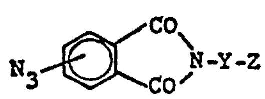

(Ib), dans lesquelles

Y représente un radical alkylène contenant de 2 à 18 atomes de carbone, substitué ou non, un radical phénylène, naphtylène, biphénylylène, cyclohexylène ou dicyclohexylméthane substitué ou non, ou un groupement

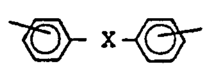

que est substitué ou non et dans lequel

X représente —O—, —S—, $—SO_2—$, $—CH_2—$, $—\underset{\substack{|\\CH_3}}{CH}—$, $—\underset{\substack{|\\CH_3}}{\overset{\substack{CH_3\\|}}{C}}—$ ou —CO—, et

Z représente un groupe $—NH_2$, —NH-alkyle dont l'alkyle a de 1 à 4 atomes de carbone, —OH, —COOH, —COCl, $—O—CO—CH{=}CH_2$, $—O—CO—\underset{\substack{|\\CH_3}}{C}{=}CH_2$, $—COO—CH{=}CH_2$ ou $—O—CH{=}CH_2$.

2. Composé de formule Ia ou Ib selon la revendication 1 dans lequel Y représente un radical alkylène en $C_2$—$C_{18}$ non substitué ou porteur d'un ou deux substituants pris dans l'ensemble constitué par le phényle, les cycloalkyles en $C_5$—$C_8$ et les aralkyles en $C_7$ et $C_8$, un radical phénylène, naphtylène, biphénylène, cyclohexylène ou dicyclohexylméthane non substitué ou portant, par noyau, un atome d'halogène, un alkyle en $C_1$—$C_4$, un cycloalkyle en $C_5$—$C_7$ ou un aralkyle en $C_7$ ou $C_8$, ou représente un radical

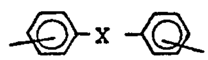

et Z et X ont les significations données au-dessous des formules Ia et Ib.

3. Composé de formule Ia ou Ib selon la revendication 1, dans lequel le groupe $—N_3$ se trouve, sur le noyau benzénique, en position ortho par rapport au groupe carboxy ou carbamoyle ou à un groupe carbonyle.

4. Composé de formule Ia ou Ib selon la revendication 1 dans lequel Y représente un radical alkylène non substitué contenant de 2 à 18 atomes de carbone, en particulier de 2 à 16, ou un radical cyclohexylène, naphtylène ou phénylène et Z représente $—NH_2$, —COOH, —COCl, —OH, $—O—CO—CH{=}CH_2$ ou $—O—CO—\underset{\substack{|\\CH_3}}{C}{=}CH_2$ .

5. Composé selon la revendication 1 qui répond à la formule VII

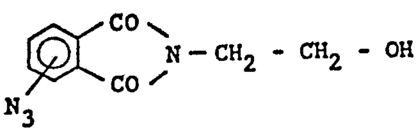 (VII),

de préférence le N-(β-hydroxyéthyl) azido-3 phtalimide.

6. Composé selon la revendication 1 qui répond à la formule VIII

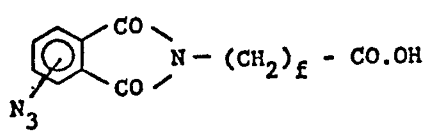 (VIII),

dans laquelle f est un nombre de 2 à 5, de préférence le nombre 5, plus particulièrement l'acide (azido-3 phtalimidyl)-6 hexanoïque.

7. Composé selon la revendication 1 qui répond à la formule IX

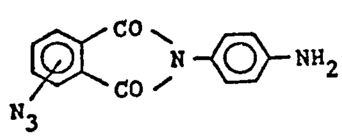 (IX),

de préférence 1 (azido-3 phtalimidyl)-4 aniline.

8. Composé selon la revendication 1 qui répond à la formule X

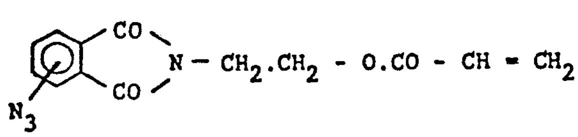 (X).

9. Composé selon la revendication 1 qui répond à la formule XI

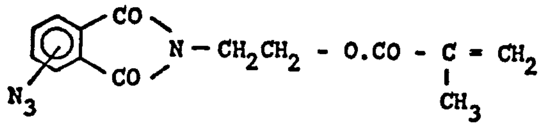

(XI).

10. Procédé de préparation d'un composé de formule Ib selon la revendication 1, procédé caractérisé en ce qu'on fait réagir un composé répondant à la formule II

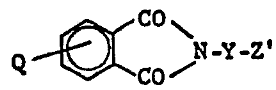

(II)

dans laquelle Y a la signification donnée sous la formule Ib, Z' représente un groupement correspondant à Z mais n'est pas —COCl, et Q représente un atome d'halogène ou le groupe nitro, dans un solvant organique inerte, à une température comprise entre environ 0 et 120°C, avec un azoture répondant à la formule III

$$M^{+n}\ (N_3^-)_n \qquad \text{(III)}$$

dans laquelle n désigne le nombre 1 ou 2 et M un cation de métal alcalin ou de métal alcalinoterreux ou un cation d'ammonium quaternaire, et, le cas échéant, on transforme le composé obtenu de formule Ib dans lequel Z représente —COOH, par traitement avec un agent de chloration, en le chlorure d'acide correspondant.

11. Procédé selon la revendication 10, caractérisé en ce qu'on utilise un composé de formule II dans lequel Q représente le groupe nitro.

12. Procédé selon la revendication 10, caractérisé en ce qu'on utilise, comme azoture de formule III, une azoture de métal alcalin, en particulier l'azoture de sodium.

13. Procédé selon la revendication 10, caractérisé en ce qu'on effectue la réaction à une température comprise entre environ 50 et 90°C.

14. Procédé pour préparer un composé de formule Ia ou Ib selon la revendication 1 dans lequel Z n'est pas le radical $—OCH=CH_2$, procédé caractérisé en ce qu'on fait réagir un composé de formule IV

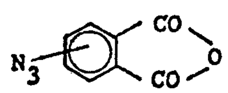

(IV)

avec une amine de formule V $H_2N—Y—Z''$ (V)

de manière à obtenir un composé de formule VI

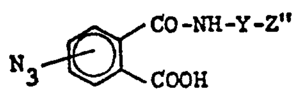

(VI)

formules dans lesquelles Y a la signification donnée au-dessous des formules Ia et Ib et Z'' représente $—NH_2$, —NH-alkyle à alkyle en $C_1—C_4$, —OH, —COOH, $—O—CO—CH=CH_2$ $—O—CO—\underset{CH_3}{C}—CH_2$ ou $—COO—CH=CH_2$, puis on cyclise éventuellement le composé de formule VI et, si on le désire, on transforme un composé ainsi obtenu de formule Ib dans lequel Z représente —COOH, par traitement avec un agent de chloration, en le chlorure d'acide correspondant.